# EUROPÄISCHE PATENTSCHRIFT

(11) **EP 2 367 469 B1**
(45) Veröffentlichungstag und Bekanntmachung des Hinweises auf die Patenterteilung: **25.10.2017**
(21) Anmeldenummer: 09804254.2
(22) Anmeldetag: 21.12.2009
(51) Int. Cl.: A61B 3/10, A61B 3/12, G01B 9/02, G01N 21/47

(54) **VORRICHTUNG UND METHODE ZUR SWEPT SOURCE OPTICAL COHERENCE DOMAIN REFLECTOMETRY**
DEVICE AND METHOD FOR SWEPT-SOURCE OPTICAL COHERENCE DOMAIN REFLECTOMETRY
DISPOSITIF ET PROCÉDÉ DE RÉFLECTOMÉTRIE OPTIQUE COHÉRENTE À SOURCE BALAYÉE

(30) Priorität: 23.12.2008 DE 102008063225
(43) Veröffentlichungstag der Anmeldung: 28.09.2011
(73) Patentinhaber: Carl Zeiss Meditec AG, 07745 Jena (DE)
(72) Erfinder: HACKER, Martin, 07745 Jena (DE); EBERSBACH, Ralf, 04626 Schmölln (DE); PABST, Thomas, 07646 Stadtroda (DE); PETERLEIN, Ulf, 07743 Jena (DE); ANTKOWIAK, Gerard, 07743 Jena (DE); BERGNER, Roland, 07745 Jena (DE); KOSCHMIEDER, Ingo, 07743 Jena (DE)
(74) Vertreter: Beck, Bernard
(86) Internationale Anmeldenummer: PCT/EP2009/009189
(87) Internationale Veröffentlichungsnummer: WO 2010/072394

(56) Entgegenhaltungen:
- EP-A1- 1 870 030
- EP-A1- 2 662 683
- WO-A1-00/16034
- WO-A1-99/60331
- WO-A1-2005/047813
- WO-A1-2006/015717
- WO-A2-2007/016296
- WO-A2-2007/065670
- WO-A2-2007/109127
- WO-A2-2007/133961
- AT-A1- 504 181
- JP-A- 2003 121 118
- US-A- 5 984 916
- US-A- 6 160 826
- US-A1- 2003 113 065
- US-A1- 2004 239 943
- US-A1- 2007 291 277
- US-A1- 2008 100 612
- US-A1- 2008 180 685
- US-A1- 2010 150 422
- US-A1- 2011 292 395
- US-B1- 6 231 186
- US-B1- 6 779 891
- US-B2- 6 930 274
- US-B2- 7 002 694
- US-B2- 7 284 861
- US-B2- 7 468 997
- US-B2- 7 982 881
- SRINIVASAN V J ET AL: "HIGH-SPEED, HIGH-RESOLUTION OPTICAL COHERENCE TOMOGRAPHY RETINAL IMAGING WITH A FREQUENCY-SWEPT LASER AT 850 NM", OPTICS LETTERS, THE OPTICAL SOCIETY, vol. 32, no. 4, 15 February 2007 (2007-02-15), pages 361-363, XP001504471, ISSN: 0146-9592, DOI: 10.1364/OL.32.000361
- LEE E C W ET AL: "In vivo optical frequency domain imaging of human retina and choroid", OPTICS EXPRESS, OSA (OPTICAL SOCIETY OF AMERICA), WASHINGTON DC, (US), vol. 14, no. 10, 15 May 2006 (2006-05-15), pages 4403-4411, XP002525498, ISSN: 1094-4087, DOI: 10.1364/OE.14.004403
- Anonymous: "Wavenumber - Wikipedia, the free encyclopedia", , 3 January 2003 (2003-01-03), XP055173124, Retrieved from the Internet: URL:http://en.wikipedia.org/wiki/Wavenumbe r [retrieved on 2015-03-03]
- LEXER F ET AL: "Wavelength-tuning interferometry of intraocular distances", APPLIED OPTICS, OPTICAL SOCIETY OF AMERICA, WASHINGTON, DC; US, vol. 36, no. 25, 1 September 1997 (1997-09-01), pages 6548-6562, XP002396007, ISSN: 0003-6935, DOI: 10.1364/AO.36.006548
- Christoph K Hitzenberger ET AL: "In-vivo intraocular ranging by wavelength tuning interferometry", Proceedings of SPIE, vol. 3251, 24 April 1998 (1998-04-24), XP055173334, ISSN: 0277-786X, DOI: 10.1117/12.306069
- YUN S H ET AL: "Motion artifacts in optical coherence tomography with frequency-domain ranging", OPTICS EXPRESS, OSA (OPTICAL SOCIETY OF AMERICA), WASHINGTON DC, (US), vol. 12, no. 13, 28 June 2004 (2004-06-28) , pages 2977-2998, XP002623685, ISSN: 1094-4087, DOI: 10.1364/OPEX.12.002977
- "Model 6200 User's Manual The External-Cavity Tunable Diode Laser", , 10 February 2000 (2000-02-10), XP055173347, Retrieved from the Internet: URL:http://assets.newport.com/webDocuments -EN/images/15220.PDF [retrieved on 2015-03-03]
- "User's Guide Model 6300-LN The Velocity Tunable Diode Laser", , 20 December 2006 (2006-12-20), XP055173355, Retrieved from the Internet: URL:http://assets.newport.com/webDocuments -EN/images/15225.PDF [retrieved on 2015-03-03]
- B Winn ET AL: "Factors affecting light-adapted pupil size in normal human subjects", Investigative Ophthalmology & Visual Science, 1 March 1994 (1994-03-01), page 1132, XP055173146, UNITED STATES Retrieved from the Internet: URL:http://www.iovs.org/cgi/content/abstra ct/35/3/1132 [retrieved on 2016-11-01]
- F. T. Arecchi: "Laser Handbook" In: "Laser Handbook", 1 January 1972 (1972-01-01), North Holland Publishing, XP055173143, ISBN: 978-0-04-410379-0 pages 1462-1469,
- Bahaa E. A. Saleh: "Fundamentals of Photonics" In: "Fundamentals of Photonics", 1 January 1991 (1991-01-01), John Wiley & Sons, XP055307856, pages 46-47,
- G. Müller: "Medical Optical Tomography: Functional Imaging and Monitoring" In: "Medical Optical Tomography: Functional Imaging and Monitoring", 1 January 1993 (1993-01-01), XP055307860, page 362,
- ZHILIN HU ET AL: "Fourier domain optical coherence tomography with a line ar-in-wavenumber spctrometer", OPTICS LETTERS, OPTICAL SOCIETY OF AMERICA, US, vol. 32, no. 24, 15 December 2007 (2007-12-15), pages 3525-3527, XP001510145, ISSN: 0146-9592, DOI: 10.1364/OL.32.003525
- TIANCHENG HUO ET AL: "Linear-in-wavenumber swept laser with an acousto-optic deflector for optical coherence tomography", OPTICS LETTERS, OPTICAL SOCIETY OF AMERICA, US, vol. 39, no. 2, 15 January 2014 (2014-01-15), pages 247-250, XP001587228, ISSN: 0146-9592, DOI: 10.1364/OL.39.000247
- Wikipedia: "Schwerpunktwellenlänge", , 17 September 2015 (2015-09-17), XP055307960, Retrieved from the Internet: URL:https://de.wikipedia.org/wiki/Schwerpu nktwellenl%C3%A4nge [retrieved on 2016-10-05]
- Changho Chong ET AL: "Spectral narrowing effect by quasi-phase continuous tuning in high-speed wavelength- swept light source References and links", OPTICS EXPRESS, vol. 16, no. 25, 5 December 2008 (2008-12-05), pages 21105-21118, XP055265280,
- T W Hensch: "Repetitively Pulsed Tunable Dye Laser for High Resolution Spectroscopy", Applied Optics, vol. 11, no. 4, 1 April 1972 (1972-04-01), pages 895-898, XP055308404,
- ZHANG JUN ET AL: "Swept laser source at 1[mu]m for Fourier domain optical coherence tomography", APPLIED PHYSICS LETTERS, A I P PUBLISHING LLC, US, vol. 89, no. 7, 16 August 2006 (2006-08-16), pages 73901-073901, XP012088545, ISSN: 0003-6951, DOI: 10.1063/1.2335405
- Izatt ET AL: "Theory of Optical Coherence Tomography", Optical Coherence Tomography: Technology and Applications, 1 January 2008 (2008-01-01), pages 47-72, XP055308414, ISBN: 978-3-540-77549-2 Retrieved from the Internet: URL:http://www.google.com [retrieved on 2016-10-06]
- NIELSEN ET AL: "Semiconductor optical amplifier based swept wavelength source at 1060nm using a scanning Fabry-Perot filter and an YDFA-based booster amplifier", OPTICS COMMUNICATIONS, NORTH-HOLLAND PUBLISHING CO. AMSTERDAM, NL, vol. 271, no. 1, 12 January 2007 (2007-01-12), pages 197-202, XP005829180, ISSN: 0030-4018
- CAHNGHO CHONG ET AL: "Large coherence length swept source for axial length measurement of the eye", APPLIED OPTICS, OPTICAL SOCIETY OF AMERICA, WASHINGTON, DC; US, vol. 48, no. 10, 1 April 2009 (2009-04-01) , pages D144-D150, XP001522873, ISSN: 0003-6935, DOI: 10.1364/AO.48.00D144
- Axsun Technology: "1060 & 1310 Swept Laser for OCT", , 1 January 2016 (2016-01-01), XP055308481, Retrieved from the Internet: URL:http://www.google.com [retrieved on 2016-10-07]
- Fujimoto ET AL: "Conference Programm Photonics West", , 1 February 2014 (2014-02-01), XP055308484, Retrieved from the Internet: URL:http://www.google.com [retrieved on 2016-10-07]
- Santec Corporation: "MEMS Based Swept Source HSL-10/20", Santec's Solutions for OCT, 1 January 2016 (2016-01-01), XP055308495, Retrieved from the Internet: URL:http://www.google.com [retrieved on 2016-10-07]
- Santec Corporation: "Optical Coherence Tomography", , 1 January 2014 (2014-01-01), XP055308497, Retrieved from the Internet: URL:http://www.google.com [retrieved on 2016-10-07]

## Beschreibung

Die Erfindung betrifft eine Vorrichtung zur Swept Source Optical Coherence Domain Reflectometry, wie sie beispielsweise in der optischen Biometrie des Auges angewendet werden kann.

Zur optimalen Anpassung einer künstlichen Intraokularlinse ist es notwendig, die optischen Verhältnisse im Auge des Patienten zu kennen, insbesondere die Abstände zwischen Hornhaut (Cornea), Augenlinse und Netzhaut (Retina).
Nachdem diese Abstandsbestimmung ursprünglich mittels Ultraschall durchgeführt wurde, ist mit dem IOL-Master von Carl Zeiss ein optisch und berührungslos arbeitendes Gerät eingeführt worden. Das Funktionsprinzip beruht dabei auf der so genannten Time Domain Optical Coherence Domain Reflectometry, einem Kurzkohärenz-Interferometrie-Verfahren, wie es beispielsweise in der WO 00/33729, auf deren Inhalt im weiteren Bezug genommen wird, beschrieben ist. Hauptbestandteil ist ein Michelson-Interferometer, welches die Detektion von Interferenzen des von Cornea, Linse und Netzhaut zurück gestreuten Lichtes ermöglicht. Durch Verwendung einer kurzkohärenten Lichtquelle können immer nur kurze Wellenzüge miteinander interferieren, wodurch die Messgenauigkeit bestimmt wird. Damit axiale Bewegungen des Patienten das Messergebnis nicht verfälschen, wird das so genannte Dual-Beam-Verfahren angewendet, bei dem das von der Cornea zurück gestreute Licht als Referenz dient.
Da der Messbereich, welcher beim Auge mehr als 43 mm betragen muss (typische Augenlängen variiert zwischen ca. 20 und 32 mm, extreme zwischen 14 und 40mm, wobei die mittlere Brechzahl ca. 1,36 beträgt), beim Michelson-Interferometer mit dem Referenzspiegel mechanisch durchfahren werden muss, dauert eine Messung üblicherweise einige Sekunden in denen der Patient z.B. nicht zwinkern darf, da der Lidschlag die Messung unmöglich machen würde.
Versuche, die Verstellgeschwindigkeit des Referenzweges zu beschleunigen, z.B. durch rotierende Prismen wie in EP 1 391 781, haben nicht zum Erfolg geführt, da die Sensitivität nicht ausreicht um die geforderte Messgenauigkeit zu erreichen.
In der DE 43 09 056 ist ein anderes auf der Kurzkohärenz basierendes Messverfahren beschrieben, bei dem Licht einer breitbandigen Lichtquelle in die Probe eingestrahlt und das aus verschiedenen Tiefen zurück gestreute Licht spektral analysiert wird. Aus einer Fourier-Transformation des detektierten Signals erhält man die Tiefeninformation. Diese Methode wird Spectral Domain OCDR (SD OCDR) oder wegen der genutzten Fourier-Transformation auch als Fourier Domain OCDR (FD OCDR) bezeichnet. Zu dieser Kategorie gehört auch die im Artikel "High-speed optical frequency-domain imaging" von S.H. Yun et al., Optics Express 2003, S. 2953 beschriebene Swept Source OCDR (SS OCDR), bei welcher die Lichtquelle spektral durchgestimmt wird und das vom Detektor aufgefangene Signal nach Fourier-Transformation ebenfalls die Tiefeninformation enthält. Die zur Realisierung von optischer Kohärenztomographie (OCT) notwendige Bildgebung wird hier, wie schon in US 5321501 für Time Domain OCT (TD OCT) gezeigt, mittels Galvo-Scannern realisiert, die den Messstrahl lateral über die Probe ablenken.
In Anlehnung an die beim Ultraschall-Messgerät eingeführte Terminologie wird die eindimensionale (axiale) Messung beim OCDR entlang der Lichtachse allgemein und damit auch im Folgenden als A-Scan bezeichnet. Ebenfalls in Anlehnung an die Ultraschallterminologie wird die zweidimensionale Messung mit einer Lateralkomponente bei OCT auch als B-scan bezeichnet.
Ein erster Versuch, SS OCDR in der optischen Biometrie anzuwenden wurde in F. Lexer, C. K. Hitzenberger, A. F. Fercher und M. Kulhavy "Wavelength-tuning interferometry of intraocular distances", Appl. Optics 36 (1997) S. 6548 - 6553 beschrieben. Diese Lösung zeigte, dass es prinzipiell möglich ist, die intraokularen Abstände im Auge zu messen, allerdings war die Messgenauigkeit mit 0,82 mm viel zu ungenau.
Eine Verbesserung dieser Lösung wurde in C. K. Hitzenberger, M. Kulhavy, F. Lexer, A. Baumgartner "In-vivo intraocular ranging by wavelenth tuning interferometry", SPIE [3251-6] 1998 offenbart. Hier wurde eine Auflösung von 0,15 mm erreicht, was aber immer noch nicht den Anforderungen entspricht. Um die Restfehler der bestimmten IOL-Refraktion auf 1/10 Dioptrien zu beschränken muss die Messgenauigkeit für die Augenlänge jedoch kleiner als 30 µm sein.
Insbesondere haben die OCDR und OCT-Verfahren an bewegten Proben, wie beispielsweise dem menschlichen Auge das Problem, dass sich die Probe während der Messung bewegen kann, was, wie in S. H. Yun et al. (2004), OPTICS EXPRESS 2977 diskutiert, die Signale deutlich verringern und verfälschen kann. Übliche Ansätze zur Behebung des Problems sind aufwendigste "tracking-methoden", bei denen die Bewegung der Probe erfasst und die Meßstrahlposition nachgeführt wird.
Solche Ansätze zur Kompensation typischer Bewegungen von einigen hundert Mikrometern pro Sekunde sind beispielsweise in Hammer et al. (2005), Journal of Biomedical Optics 10(2), 024038, sowie in US 2006/105903 beschrieben. Nachteilig ist bei solchen Ansätzen, dass, trotz des grossen technischen Aufwandes, aufgrund der endlichen Laufzeiten solcher Systeme immer gewisse Nachführungsfehler resultieren, insbesondere bei sehr schnellen Augenbewegungen, wie Sakkaden. Die WO 2005/047813 offenbart eine optische Kohärenztomografieeinrichtung, welche einen um eine Zentralwellenlänge von 1320 nm durchstimmbaren Laser aufweist und einen Tiefenmessbereich von 5,8 mm erreicht.
In der US 2008/100612 wird eine Bedienoberfläche für eine optische Kohärenztomografieeinrichtung beschrieben, ohne auf deren technische Merkmale einzugehen.
In dem Artikel "In vivo optical frequency domain imaging of human retina and choroid", E.C. W. Lee, J. F. de Boer, M. Mujat, H. Lim und S.H. Yun, Optics Express Vol. 14, Nr. 10, (2006), S. 4403-4411 wird eine optische Kohärenztomografieeinrichtung mit einem um eine Zentralwellenlänge von 1050 nm durchstimmbaren Laser beschrieben, welche einen Tiefenmessbereich von 2,4 mm an der Retina erreicht.

Ausgehend vom Stand der Technik ist es daher die Aufgabe der Erfindung, eine Vorrichtung anzugeben, mit der die intraokularen Abstände im Auge schnell und genau gemessen werden können, insbesondere auch bei Auftreten typischer Augenbewegungen, ohne die Nachteile einer aktiven Messstrahinachführung mit einer Probenbewegung, wie beispielsweise Latenzzeitfehler, aufzuweisen.
Diese Aufgabe wird gelöst durch eine Vorrichtung zur Swept Source Optical Coherence Domain Reflectometry (SS OCDR) an beweglichen Proben, insbesondere menschlichen Augen, zur Gewinnung von A-Scans mit einem Messbereich entsprechend der Probenlänge, mit einer um eine Schwerpunktswellenzahl k₀ durchstimmbaren Laser-Lichtquelle und mindestens einem Empfänger für das aus der Probe zurück gestreute Licht, wobei die Probe über eine Koppeleinrichtung mit einem Messstrahl vom Durchmesser D auf der Probenoberfläche beleuchtet wird, die Lichtquelle eine spektrale Linienbreite δk<168m⁻¹ aufweist und die Durchstimmung der Lichtquelle in τ < 44s / (D*k₀) erfolgt.
Damit wird insbesondere eine aufwandgeringe und effiziente Abstandsmessungen über die Gesamtlänge des Auge realisiert, da, trotz typischer Augenbewegungen von bis zu 1000 µm/s und bei nur moderaten Anforderungen an die an die Durchstimmrate der Quelle, störende Signalverluste infolge von Probenverschiebungen bei Abstandsmessungen zwischen Flächen der Hornhaut, Cornea und Retina vermieden werden.
Die erfindungsgemäße Lösung realisiert dazu, dass die Durchstimmzeit des Lasers so an die sinnvollen Messstrahlverläufe in der Probe angepasst ist, dass die während der Durchstimmzeit des Lasers möglichen lateralen Probenverschiebungen überwiegend nur Bruchteile des kleinsten möglichen Messstrahldurchmessers in der Probe betragen können. Damit wiederum werden störende Signalverluste durch Ausmitteln unterschiedlicher lateraler Interferenzmodulationen vermieden, da die an verschiedenen Zeitpunkten während der Durchstimmung vom Messstrahl beleuchteten Probenvolumina einen ausreichenden Überlapp besitzen.
Unter sinnvolle Messstrahlverläufen werden hier diejenigen verstanden, die für Beabstandung ausreichende Signalstärken von Hornhaut, Kristalllinse und Retina liefern können, indem sie Messstrahltaillenpositionen im Bereich zwischen Kristalllinsenrückseite kürzerer Augen bis hin zur Retina langer Augen aufweisen (8...40mm).

Gleichzeitig vermeidet die erfindungsgemäße Lösung auch eine Signalverminderung und -verfälschung durch axiale Probenverschiebungen während der Durchstimmung, wie beispielsweise Dehnung oder Kompression der A-Scans mit resultierenden inakzeptablen Fehlern in der Beabstandung der Augenstrukturen.

Es werden also überwiegend ungestörte Signale realisiert, ohne dass eine aktive Nachführung des Messstrahls mit Probenbewegungen erfolgen muss.

Dabei ist es von Vorteil, wenn die Lichtquelle einen spektralen Durchstimmbereich Δk um eine Schwerpunktswellenzahl k₀ von mindestens Δk> 18000m⁻¹ aufweist. Vorteilhafterweise ist dabei das Verhältnis von Durchstimmbereich Δk und Linienbreite δk größer als 360, weiter bevorzugt größer als 2000, weiter bevorzugt größer als 4000, noch weiter bevorzugt größer als 9000. Dieses Verhältnis stellt sicher, dass ein ausreichendes Verhältnis zwischen Messtiefe und -auflösung realisiert wird.
Ein weiterer Vorteil ergibt sich, wenn der Quotient aus Durchstimmrate (Δk / τ) und Laserlinienbreite δk größer als 18 kHz ist, bevorzugt auch größer als 4 MHz, weiter bevorzugt größer als 40 MHz.
Dabei ist es besonders vorteilhaft, wenn die Detektion des von Cornea, Augenlinse und Retina zurück gestreuten Lichtes während einer einzigen Durchstimmung der Lichtquelle erfolgt. Dabei ist es günstig, wenn die Digitalisierung des am Empfänger detektierten zurück gestreuten Lichts mit einer Rate von mehr als Δk / (τ*δk) erfolgt. Damit wird sichergestellt, dass eine ausreichende Abtastung der spektralen Informationen erfolgt.

Besonders geeignet ist die erfindungsgemäße Vorrichtung, wenn die Linienbreite δk der Lichtquelle zwischen 22 und 50 m⁻¹ liegt. Solche Linienbreiten können beispielsweise mit durchstimmbaren Faserringlasern realisiert werden und bieten akzeptable Sensitivitätsabfälle über die Messtiefe.
Es ist vorteilhaft, wenn die Bandbreite des mindestens einen Empfängers, beispielsweise beschrieben durch die Grenzfrequenz mit 3 db Signalabfall, größer als 2 * Δk / (τ*δk) und bevorzugt kleiner als 80 MHz ist.

Erfindungsgemäß ist in der Vorrichtung zur SS OCDR am Auge eine durchstimmbare Laserquelle, ein Interferometer mit einem Referenzarm und einem von der Probe abgeschlossenen Probenarm, Detektoren an den Interferometerausgängen sowie eine Signalverarbeitungseinheit für die detektierten Signale vorgesehen Erfindungsgemäß sind in der Vorrichtung zur SS-OCDR am Auge vorzugsweise die Lage von Retina- und Comea-Signalen im A-Scan und die Laserlinienbreite δk aneinander angepasst. Besonders bevorzugt wird auch ein Referenzinterferometer zur Wellenzahlreferenzierung der Quelle und damit zur Längenkalibrierung des OCDR-Signals verwendet.

Für die Vorrichtung zur SS OCDR am Auge ist der Messstrahldurchmesser D im Bereich des Probeneintritts kleiner als 3 mm. Auch kann bevorzugt der Messstrahl vor dem Eintritt in das Auge konvergent sein, wobei die Größe der Konvergenz vorzugsweise einstellbar oder umschaltbar ist. Insbesondere kann durch die Einstellung der Konvergenz die relative Stärke der detektierten Signals aus verschiedenen Augenbereichen (Retina, Linse, Cornea) zueinander angepasst oder optimiert werden, um eine gemeinsame Verarbeitung zur Abstandsmessung und visuellen Bewertung der Signale zu erleichtern.
Alternativ kann es auch von Vorteil sein, wenn der Messstrahl vor dem Eintritt in das Auge kollimiert ist und Mittel zur Umfixierung des Auges vorgesehen sind um spekulare Cornea- und Linsensignale detektieren zu können. Durch das Umfixieren kann auf der optischen Achse des Auges gemessen werden, welche von der Sehachse um bis zu 14° abweichen kann. Es ist auch günstig, wenn zwischen kollimierten und konvergentem Messstrahl umgeschaltet werden kann.
Eine weitere günstige Realisierung der Erfindung ergibt sich, wenn der Messstrahl außerhalb des Cornea-Apex auf das Auge trifft, wobei vorzugsweise eine Einrichtung zur Positionierung des Messstrahles relativ zum Auge vorgesehen ist, welche insbesondere durch Auswertung des vom Empfänger detektierten Lichts angesteuert werden kann. Damit wird verhindert, dass der starke Reflex vom Cornea-Scheitel die Detektionseinrichtungen sättigt oder auch das Signal-Rausch-Verhältnis verschlechtert wird, beispielsweise durch die Erhöhung des von dem starken Comea-Reflex verursachten Schrotrausch-Anteils.
Eine solche Einrichtung zur Positionierung des Messstrahles, insbesondere synchronisiert zur Durchstimmung des Lasers, erlaubt die Gewinnung von B-Scans und somit die Realisierung von OCT über das gesamte Auge. Derartige Positionierungen des Messstrahles können beispielsweise durch an sich bekannte optische Konfigurationen in Kombination mit Winkelablenkungen durch ebenfalls bekannte Galvanometer-Scannern (US 5321501) erfolgen.

Besonders vorteilhaft ist es, wenn in der Durchstimmzeit τ der Lichtquelle ein Photonenfluss von 3 * 10⁸ bis 1 * 10¹³ auf die Probe gerichtet wird. Durch geeignete Wahl der Wellenlänge (z. B. 1060nm) können phototoxische Effekte vermieden werden. Damit kann eine hohe Empfindlichkeit für die schwach streuenden Augenstrukturen durch Optimierung des schrotrauschbedingten Signal-Rausch-Verhältnisses realisiert werden, ohne dass das Augengewebe geschädigt wird.

Es kann weiterhin vorteilhaft sein, wenn die Messung entlang der Sehachse des Auges erfolgt, da insbesondere die Abstandsinformationen auf dieser Achse für die Anpassung von Intraokularlinsen von größtem Nutzen sind. Dazu sollten Messstrahl und Fixierlicht kollinear auf das Auge treffen, können dabei aber auch verschiedene Divergenzen aufweisen, beispielsweise um einen möglichen Refraktionsfehler des Auges zu kompensieren.

Eine vorteilhafte Ausgestaltung der Erfindung ergibt sich, wenn in Referenzarm und/oder Quellarm und/oder Detektionsarm und/oder Referenzinterferometer Monomodefasern verwendet werden, um störende parasitäre Interferenzen zwischen verschiedenen sich in der Faser ausbreitenden Moden und sich daraus ergebende Artefakte zu vermeiden. Gleichfalls sind die offenen Faserenden als schräg polierte Flächen ausgelegt, um störende Rückreflexe zu vermeiden.

Vorteilhaft ist, wenn Referenzsignale und Probensignale mit konstanten Abtastraten digitalisiert werden, wobei bevorzugt gleiche Abtastraten für Referenz- und Probensignale verwendet werden. Zur Verringerung des auftretenden Datenvolumens kann aber auch bei geeigneter Wahl des Referenzinterferometers die Abtastung des Referenzsignals mit geringerer Abtastrate als die Abtastung des Probensignals erfolgen. Der Messstrahl weist eine Wellenlänge zwischen 600 und 1150 nm auf, wobei Wellenlängen von 700 nm, 800 nm und 1060 nm besonders bevorzugt sind.
Eine bevorzugte Ausgestaltung der Vorrichtung zur SS OCDR am Auge ergibt sich dadurch, dass eine Einrichtung zur Projektion von Zielmarken mit einer Wellenlänge zwischen 400 und 1500 nm auf das Auge, insbesondere die Cornea, und eine Beobachtungseinheit zur Erfassung der Reflexe dieser Zielmarken vorgesehen ist. Diese können auch hinsichtlich Lage- und Formbestimmung von Hornhaut und Linse ausgewertet werden.
Weiter vorteilhaft ist es dabei, wenn die Beobachtungseinheit, beispielsweise eine Kamera, zur Überprüfung der Justage des Messstrahles relativ zum Auge vorgesehen ist, wobei die Beobachtungseinheit bevorzugt für die Wellenlängen des Messstrahls und der Zielmarken empfindlich ist. Hier erscheinen Kameras mit einem Siliziumsensor wegen ihrer ausreichenden Restempfindlichkeit im nahen Infrarot besonders geeignet.
Eine besonders geeignete Ausgestaltung der Erfindung ergibt sich wenn die Lichtquelle gegenüber dem Auge beweglich ist, wobei bevorzugt Lichtquelle und Referenzinterferometer fest verbunden sind.
Eine andere vorteilhafte Bedingung für die Realisierung der Erfindung besteht darin, dass das Interferometer gegenüber dem Auge beweglich ist, wobei bevorzugt Lichtquelle und Interferometer fest verbunden sind.
Dabei ist von Vorteil, wenn die elektrischen und optischen Verbindungen trennbar gestaltet sind.
Mit der erfindungsgemäßen Vorrichtung ist es erstmals möglich, ein gesamtes Auge mit Hilfe des OCDR-Verfahrens in einem A-Scan mit einer Genauigkeit von besser 100 µm, insbesondere besser als 30 µm, zu vermessen, um so Messwerte zur Anpassung einer Intraokularlinse zu gewinnen. Die Messung kann hierbei zwei oder mehr gleichzeitige Abstandsmessungen zwischen Cornea, Linse und Retina umfassen und ist robust gegenüber üblichen axialen und lateralen Patientenbewegungen, welche typisch im Bereich von 1 mm/s liegen.

Die Erfindung wird im Folgenden an Hand der Zeichnungen näher erläutert.

Es zeigen
Fig. 1 einen grundsätzlichen Aufbau der erfinderischen Vorrichtung
Fig. 2 eine bevorzugte Ausführungsform der Erfindung
Fig. 3 verschiedene Lösungen zur Anordnung der Referenzebene für die Messung

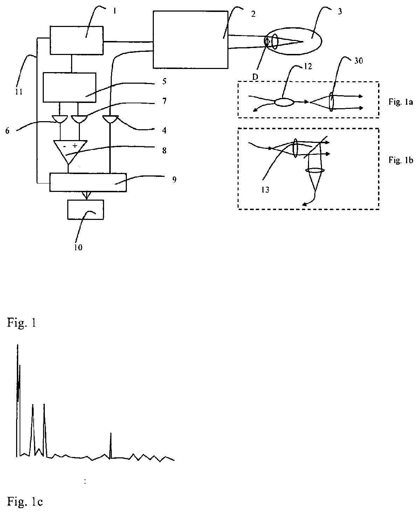

Der grundsätzliche Aufbau zur Realisierung der Erfindung in Fig. 1 besteht aus einem geeigneten durchstimmbaren Laser 1, welcher durch die Größen Durchstimmzeit τ, Wellenlänge λ, spektraler Durchstimmbereich Δk, Schwerpunktswellenzahl k₀ und Laserlinienbreite δk charakterisiert ist.
Eine Strahlformungs- und Kopplungseinheit 2 dient sowohl dazu den Strahl des Lasers 1 auf die Probe 3 (hier schematisch als Auge dargestellt) zu richten als auch das von der Probe 3 zurückgestreute Licht einem Detektor 4 zuzuführen, D ist dabei der Durchmesser des Messstrahls beim Auftreffen auf die Probe (hier Hornhaut des Auges). Dem Laser 1 ist ein Referenzinterferometer 5 zugeordnet, dessen bevorzugt zwei Detektoren 6, 7 über einen Differenzverstärker 8 mit einer Datenerfassungseinrichtung 9 verbunden sind. Die Detektoren bestehen aus InGaAs-Photodioden mit Chipdurchmessern > 0.1mm und Bandbreiten <80MHz.
Das Referenzinterferometer 5 ist hierbei ein faseroptisches Mach-Zehnder-Interferometer mit zwei mit den Detektoren 6 und 7 verbundenen Ausgängen. Die optische Weglängendifferenz zwischen den Armen des Referenzinterferometers ist LREF. Die Arme des Referenzinterferometers 5 können aus Fasern mit verschiedenen chromatischen Dispersionen bestehen, so dass trotz Weglängendifferenz gleiche Dispersion in beiden Armen realisiert wird, um einen maximalen Modulationskontrast an den Detektoren 6 und 7 zu erzielen. Prinzipiell ist auch eine Signalerfassung mit nur einem Detektor 6 und ohne Differenzverstärker 8 bei verminderter Signalqualität möglich.
Die Datenerfassungseinrichtung 9 ist ebenfalls mit dem Detektor 4 für das von der Probe 3 zurückgestreute Licht verbunden, sowie mit der Steuer- und Auswerteeinheit 10. Diese Verbindung erlaubt die Erfassung und Verarbeitung der vom Detektor 4 empfangenen und mit der Datenerfassungseinrichtung 9 aufgezeichneten Signale mit der Steuer- und Auswerteeinheit 10, sowie Synchronisation des Messablaufs zwischen Datenerfassungseinrichtung 9 und Steuer- und Auswerteeinheit 10. Des Weiteren ist eine Synchronisationsverbindung 11 zwischen Datenerfassungseinrichtung 9 und Laser 1 vorhanden, mit deren Hilfe die Durchstimmung des Lasers 1 mit der Datenerfassung synchronisiert wird. Die Datenerfassungseinrichtung 9 ist hierbei auch in der Lage Steuersignale zum Laser 1 zu senden oder von diesem zu empfangen, falls der Laser eine periodische Durchstimmung eigenständig durchführt.

Dabei kann die Strahlformungs- und Kopplungseinheit 2 wie in Fig. 1a dargestellt mit einem Faserkoppler 12 aufgebaut sein, alternativ ist auch die Realisierung mit einem Strahlteiler 13 wie in Fig. 1b möglich.
Die Steuer- und Auswerteeinheit 10 steuert die Durchstimmung des Lasers 1 über die Datenerfassungseinrichtung 9 (mit spektralem Durchstimmbereich Δk in der Durchstimmzeit τ) und das von der Probe 3 zurückgestreute und vom Detektor 4 gemessene Licht wird digitalisiert und in an sich bekannter Art und Weise zur Rekonstruktion des A-Scans einer Fourier-Transformation, beispielsweise einer Diskreten Fourier-Transformation DFT unterzogen.
Dabei erfolgt die Rekonstruktion des A-Scans insbesondere derart, dass der Stützstellenabstand im A-Scan kleiner ist als 4*ln2/Δk, insbesondere kleiner als 4/3*ln2/Δk. Enthält das Koppelelement 2 keine weiterer Einrichtung zur Erzeugung eines Referenzlichtanteils wird damit als A-Scan ein Autokorrelationssignal gewonnen, ähnlich dem in K. Hitzenberger, A. F. Fercher und M. Kulhavy "Wavelength-tuning interferometry of intraocular distances", Appl. Optics 36 (1997) S. 6548 - 6553 beschriebenen. Dieses entlang der Lichtausbreitungsachse um den stärksten Signalanteil (meist Hornhautoberflächensignal) symmetrische A-Scan kann so beschnitten werden, dass redundante Informationen entfallen (Fig.1c).
Das Referenzinterferometer 5 dient dabei der exakten Bestimmung des zeitlichen Ablaufs des Durchstimmprozesses. Eine gleichzeitige Aufzeichnung von Referenzinterferometer- und Messsignal mit der Datenerfassungseinrichtung 9 erlaubt dazu eine sehr genaue Zuordnung der momentanen, relativen Wellenzahlen des Lasers zu den Messsignalen. Insbesondere können darüber Messsignale für beliebige Stützpunktsysteme im Spektralraum interpoliert werden ("re-mapping"), beispielsweise bezüglich äquidistanter Wellenzahlen in dispersiven Geweben um nach der Fourier-Transformation A-Scans mit konstanter Auflösung über den gesamten Messtiefenbereich zu erhalten. Weiter ist damit auch eine numerische Kompensation der die Auflösung vermindernden chromatischen Dispersion möglich.
Ein solches re-mapping und diese Dispersionskompensationen werden in US 7330270 für SD-OCT beschrieben, auf deren kompletten Inhalt hier Bezug genommen wird.

Die Gewinnung äquidistanter Wellenzahlen kann, wie in V.J. Srininivasan et al Optics Letters 32, 361 beschrieben, dabei beispielsweise über eine Phasenrekonstruktion der Signale aus dem Referenzinterferometer 5 mittels Hilbertransformation mit nachfolgender Bestimmung äquidistanter Phasenabstände erfolgen.
Dazu ist weiter sicherzustellen, dass statistische Fehler bei der Durchstimmung der Wellenzahl der Laserquelle 1 so klein sind, dass die Phasen der Signale aus dem Referenzinterferometer 5 eindeutig rekonstruiert werden können. Insbesondere soll gelten, dass die Standardabweichung des Wellenzahlfehlers σₖ unter einer durch die Länge LREF vorgegebene Grenze σₖ<π/(LREF*v) bleibt, so dass Phasenfehler > n beispielsweise nur alle 400 (v=4.05) ... 10000 (v=3.23) Abtastungen auftreten können.
Die OCDR-Signale können auch durch Mittelungen und Abzug von Untergrundsignalen verbessert werden, die ohne Probe oder bei ausgeblendetem Messstrahl gewonnen werden können.
Fig. 1c zeigt für das Beispiel eines Auges als Probe 3 den erhaltenen A-Scan, d.h. das Längsprofil entlang der Lichtachse. Die Spitzen zeigen dabei von links nach rechts die Reflexe von Hornhaut (Cornea), Linsenvorderseite, Linsenrückseite und Netzhaut (Retina).

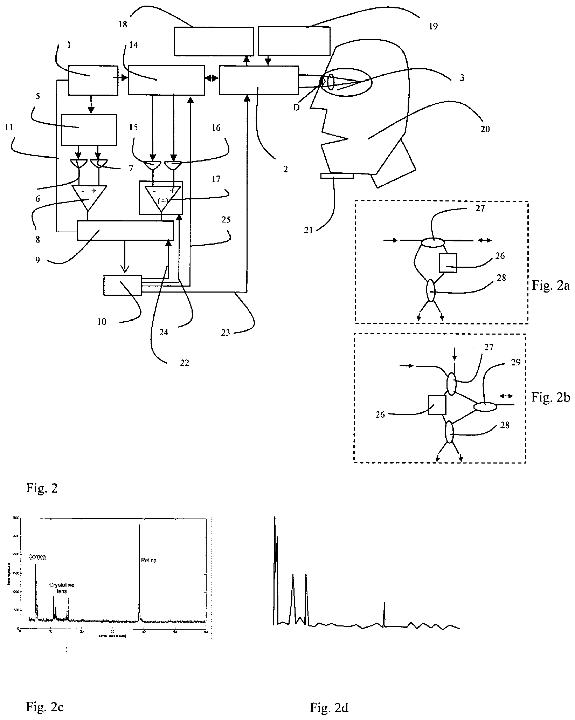

Fig. 2 zeigt eine bevorzugte Ausführungsform der Erfindung zur Biometrie des Auges. Der Grundaufbau aus durchstimmbarer Laserquelle 1, Strahlformungs- und Kopplungseinheit 2, Referenzinterferometer 5 mit den Detektoren 6, 7, dem zugeordneten Differenzverstärker 8 und der Datenerfassungseinheit 9 entspricht dem aus Fig. 1 bekannten Aufbau. Zusätzlich ist dem Laser 1 ein weiteres Interferometer 14 nachgeordnet, welches eine Interferenz von in die Probe 3 (Auge) eingestrahltem und zurückgestreutem Licht mit einem Referenzlichtanteil realisiert, die dann mit den Detektoren 15 und 16 detektiert werden können. Den beiden Detektoren 15, 16 des Interferometers 14 ist ein Verstärker 17 zugeordnet, welcher in einer besonderen Ausführungsform zwischen Differenz- und Summenverstärkung umschaltbar ist. Der Ausgang des Verstärkers 17 ist wiederum mit der Datenerfassungseinheit 9 verbunden.

Die grundsätzliche Signalverarbeitung, also beispielsweise die Erfassung der Signale von Referenzinterferometer 5, das "Re-mapping", der Untergrundabzug und die Fourier-Transformation, erfolgt hierbei in gleicher Weise wie zuvor für die Autokorrelationssignalgewinnung beschrieben.

Der Referenzlichtanteil wird dabei mittels eines transmissiven Referenzarmes in einem aus faseroptischen Komponenten bestehenden Interferometer 14 erzeugt. Alternativ können auch reflektiv, beispielsweise an Faserenden, gewonnene Referenzlichtanteile genutzt werden. Fig. 2a und 2b zeigen zwei Varianten eines solchen Faserinterferometers. Im Referenzarm dieser Interferometer wird weiter ein einstellbarer Abschwächer 26 verwendet, der eine Einstellung des Referenzlichtanteils zur günstigen Aussteuerung der Detektoren 15 und 16 erlaubt. Nicht dargestellt sind möglich Polarisationskompensatoren in Proben- oder Referenzarm, die einen partiellen oder vollständigen Abgleich der Polarisationszustände des Referenz- und Probenlichtes zur ausreichenden Interferenz ermöglicht.

Der erster Faserkoppler 27 dient zur Aufteilung in ein Proben- und Referenzarm.

Dabei wird in Fig. 2a das Teilungsverhältnis des Faserkopplers 27 so eingestellt, dass der überwiegende Teil des von der Probe zurückkommenden Lichtes den Detektoren 15 und 16 zugeführt wird.
Der Faserkoppler 28 dient zur interferometrischen Überlagerung des rückkehrenden Probenlichtes mit dem Referenzlichtes aus dem Referenzarm und der Zuführung der gegenphasigen Signalanteile zu den Detektoren 15 und 16 für balancierte Detektion. Prinzipiell können auch die in WO 2004/111 661 beschriebenen Möglichkeiten zur Bestimmung weiterer Quadraturkomponenten zur Spiegelartefaktunterdrückung in FD-OCDR zum Einsatz kommen.
In Fig. 2b wird ein Teil der Referenzarmabschwächung durch den Faserkoppler 27 bewirkt und Faserkoppler 29 wird derart gewählt, dass von der Probe zurückkehrendes Licht überwiegend den Detektoren 15 und 16 zugeführt wird. Dies ist Vorteilhaft, wenn der einstellbare Abschwächer 26 nur einen begrenzten Abschwächungsbereich aufweist. Weiterhin kann über den Faserkoppler 27 noch sichtbares Licht von der Fixierlichtquelle 19 eingespeist werden.
Ist der einstellbare Abschwächer 26 so ausgelegt, dass das Referenzlicht auch vollständig blockiert werden kann, so können damit auch die oben erwähnten Autokorrelationssignale gewonnen werden. In diesem Falle ist der Verstärker 17 auf Summation umzuschalten, während er bei Nutzung des Referenzlichtanteils für eine balancierte Detektion auf Differenzbildung zu schalten ist. Summensignale bei nichtblockiertem Referenzarm können auch zur Laserüberwachung genutzt werden.

Für die Vermessung des Auges hat es sich bewährt eine Messwellenlänge λ im Infrarot-Bereich zu wählen, also z.B. zwischen 600 und 1150 nm, wobei Werte wie 700 nm, 800 nm und 1060 nm besonders bevorzugt sind.
Des Weiteren verfügt die Anordnung über eine Beobachtungskamera 18 und eine Fixationsquelle 19. Die Beobachtungskamera 18 entspricht der CCD-Kamera in der WO 00/33729, auf deren kompletten Inhalt hiermit Bezug genommen wird und dient wie dort der Kontrolle der Ausrichtung der Messanordnung zum Auge 3 des Patienten, dazu können auch zusätzliche Zielmarken auf das Auge projiziert werden. Dafür sollte sie für die Messwellenlänge λ zumindest eine Restempfindlichkeit aufweisen, was von Kameras mit Siliziumsensor (auch in CMOS-Ausfertigung) im Allgemeinen gegeben ist, insbesondere der vergleichsweise starke Reflex des Messstrahls an der Hornhaut ist gut erkennbar.
Die Fixationsquelle 19 dient der Ausrichtung des Auges zur Messanordnung und kann beispielsweise analog dem Vorschlag in DE 103 23 920, auf deren kompletten Inhalt hiermit Bezug genommen wird, aufgebaut sein. Durch entsprechende Ansteuerung kann der Patient so beeinflusst werden, dass wahlweise auf der Augenachse oder der Sehachse gemessen wird ("Umfixierung").
Zur Messung wird legt der Patient seinen Kopf 20 an die Probandenaufnahme 21 um diesen weitgehend ruhig zu halten. Laser 1 und Interferometer 14 sind lagestabil miteinander verbunden und können gemeinsam mittels hier nicht dargestellter Mittel zur Justierung bzgl. des Patientenauges 3 verschoben werden. Diese lagestabile Verbindung erweist sich auch als vorteilhaft wenn die Lichtleitung über Fasern, insbesondere Mono-Mode-Fasern ausgeführt wird. Insbesondere kann damit auf den Einsatz veränderbarer faseroptischer Polarisationskompensatoren ("paddles") verzichtet werden.

Die Datenerfassungseinheit 9 erlaubt die Digitalisierung von Signalen vom Verstärker 8 ( Signal vom Referenzinterferometer 5) und vom Verstärker 17 (Signal vom Interferometer 14) mit verschiedenen Bittiefen.
Zur Reduzierung des zu übertragenden Datenvolumens wird dabei die Mindestbittiefe **MBT** für die Digitalisierung des Referenzinterferometersignals an die optische Weglängendifferenz **LREF** im Referenzinterferometer 5 und den optischen OCDR-Messbereich **ZMAX** folgendermaßen angepasst: MBT>=log2 (2/(1-|cos(π*LREF/2ZMAX)|). Bei einem LREF=100mm und einem ZMAX=60mm ergibt sich MBT = 4bit.

Für die Digitalisierung der OCDR-Signale vom Interferometer 14 werden Bittiefen von <14bit verwendet, insbesondere ist eine Bittiefe von 10bit ausreichend.

Die Steuereinheit 10 ist über einen Leitung 22 mit der Datenerfassungseinheit 9 verbunden um eine Umschaltung der Bittiefe des gemessenen Signals zu ermöglichen. Eine solche Bittiefenumschaltung ist beispielsweise für eine optimale Aussteuerung bei Umschaltung zwischen Autokorrelationsmessung und Messung mit Referenzarm vorteilhaft.

Weiterhin ist die Steuereinheit 10 mit der Strahlformungs- und Kopplungseinheit 2 verbunden (Leitung 23) um eine Umschaltung des Fokus (wie in DE 103 23 920) realisieren zu können, über eine weitere Leitung 24 kann der Verstärker 17 gesteuert werden, insbesondere zwischen Summen- und Differenzfunktion umgeschaltet werden. Zusätzlich erfolgt eine Umschaltung der Verstärkungsverhältnisses des Verstärkers insbesondere bei Übersteuerung der OCDR-Signale oder bei Umschaltung zwischen Autokorrelationsmessung und Messung in Bezug auf einen Referenzlichtanteil aus einem Referenzarm

Zur Fokussierung enthält die Koppeleinheit 2 eine Fokussierlinse 30, die im Auge einen Messstrahlfokus posterior zum vorderen Augenabschnitt (bevorzugt 8 bis 25mm hinter der Cornea, maximal 40mm hinter der Cornea) erzeugt. Alternativ können auch diffraktive oder reflektive Fokussierelemente genutzt werden. Dabei kann auch eine Anpassung an die Refraktion des Auges mittels einer geeigneten Anpassungsoptik erfolgen.
Dabei ist es vorteilhaft, wenn die Einspeisung der Fixationsquelle 19 in die Koppeleinheit 2 derart erfolgt, dass die Fixation durch die Fokussierung des Messstrahles nicht beeinflusst wird und eine möglichst gute Abbildung des Fixierlichtes auf der Retina erfolgt, also bevorzugt hinter der Fokussierlinse 30.
Weiterhin kann die Koppeloptik auch eine Einrichtung zur Verschiebung oder Ablenkung des Messstrahles beinhalten, insbesondere um OCDR-Signale zu verbessern, beispielsweise indem störende starke Hornhautreflexe durch laterale Messstrahlverschiebung vermieden werden.

Die Umschaltung der Abschwächung bzw. Blockierung des Referenzarmes im Interferometer 14 erfolgt über eine Verbindung 25 zwischen Steuereinheit 10 und Interferometer 14. Fig. 2c zeigt einen A-Scan, der mit einer Anordnung nach Fig. 2 (mit Referenzarm) gemessen wurde, Fig. 2d eine analoge Messung mit blockiertem Referenzarm, d.h. mittels Bestimmung der Autokorrelationsfunktion des rückgestreuten Probenlichtes ohne Darstellung des spiegelsymmetrischen Signalsanteils.

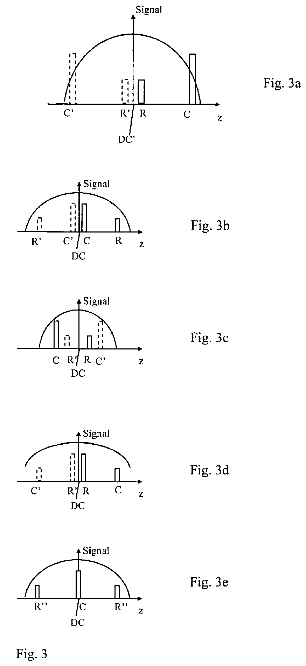

Mit den bevorzugten Werten Durchstimmbereich Δk=112000m⁻¹, D=2 mm, Wellenlänge λ=1060nm und Durchstimmzeit τ = 500µs lässt sich erstmalig die gesamte Augenlänge und die Lage der Augenlinse in einem Messvorgang mit einer OCDR-Auflösung/Messgenauigkeit von <30 µm bestimmen. Dabei ist gesichert, dass das Messergebnis nicht durch unwillkürliche Augenbewegungen verfälscht ist.
Es hat sich gezeigt, dass die maximale Laserlinienbreite δk vom Messregime abhängt. Für den Fall der Messung der Autokorrelationsfunktion, d.h. mit blockiertem Referenzarm muss die Laserlinienbreite kleiner als 162 m⁻¹ sein.
Falls eine Anordnung mit Referenzarm verwendet wird kann durch geeignete Festlegung der Referenzebene in der Probe erreicht werden, dass 1. die Signale von Cornea, Linse und Retina mit ausreichender Stärke erfasst werden und 2. Spiegelartefakte unterdrückt bzw. identifiziert und herausgerechnet werden können. Fig. 3 zeigt dieses schematisch für verschiedene Lagen der Referenzebene, welche über die Weglängendifferenzen zwischen Referenz- und Probenlichtpfad einstellbar ist.
In Fig. 3a wurde die Referenzebene hinter die Retina (R) eingestellt, es ergibt sich eine maximale Laserlinienbreite von 93 m⁻¹ (Signalabfall von 80dB über einen Gesamtmessbereich von 54mm, schematisch als Kurve dargestellt). R', C' bezeichnen hier und im Folgenden die Spiegelartefakte.
In Fig. 3b ist die Referenzebene vor die Cornea (C) eingestellt, es ergibt sich eine maximale Laserlinienbreite von 81 m⁻¹ (möglicher Signalabfall von 60dB über einen Gesamtmessbereich von 54mm, da die Cornea besser reflektiert als die Retina).
In Fig. 3c wurde die Referenzebene zwischen Cornea (C) und Retina (R) eingestellt, es ergibt sich eine maximale Laserlinienbreite von 162 m⁻¹.
In Fig. 3d wurde wie in Fig. 3a die Referenzebene hinter die Retina (R) eingestellt, mit einem angestrebten Signalabfall von nur 20dB über einen Gesamtmessbereich von 54mm ergibt sich eine maximale Laserlinienbreite von 47 m⁻¹. Dies ist die bevorzugte Laserlinienbreite δk. Hierbei muss ein Mindestabstand von 64mm zwischen der Referenzeben und dem dem Auge am nächsten liegenden, vom Messstrahl durchquerten optischen Element realisiert werden.
Fig. 3e zeigt die Verhältnisse im Fall der Messung der Autokorrelationsfunktion.
Bei deutlich schmaleren Laserlinienbreiten (<20 m⁻¹) besteht die Gefahr, dass Signalartefakte durch Reflexe von den optischen Elementen auftreten und die Interpretation der Messergebnisse verfälschen könnten.

Sollen Mess- und Spiegelsignal gemeinsam zur Auswertung herangezogen werden (Fig. 3c) oder besteht die Gefahr eines unerwünschten Überlappens zwischen Messsignalen (z.B. R, C in Fig. 3a, b), so ist es zur Fehlervermeidung eine eindeutige Identifizierung der Spiegelsignale (R', C') notwendig.
Bevorzugt werden hierfür Variationen der Mess- oder Rekonstruktionsbedingungen derart realisiert, dass eindeutige Veränderungen im Spiegelsignal erfolgen, die eine manuelle und automatisierte Identifizierung oder auch Unterdrückung erlauben.
Eine erste bevorzugt Möglichkeit hierfür ist die Variation der Differenz zwischen Referenz- und Probenarmlänge, welche eine zum Messsignal entgegengesetzte Ortsvariation beim Spiegelsignal bewirkt, die numerisch detektiert werden kann. Dies kann beispielsweise durch Variation des Abstandes zwischen Proband und Messgerät erfolgen, bevorzugt im Bereich 0.1 bis 4mm, und durch Bestimmung des ersten Momentes der Signalverteilung des zu identifizierenden Signalanteils. Alternativ können auch zwischen aufeinanderfolgenden Durchstimmungen des Lasers sehr schnell Phasenverschiebungen zwischen Referenz- und Probenarm erfolgen, mittels derer aus den Spektraldaten ein komplexes FD-OCDR Signal rekonstruiert werden kann, bei dem auch das Spiegelartefakt ganz oder teilweise unterdrückt sein kann (vgl. US 7433046 B2). Diese vollständige oder partielle Spiegelartefaktunterdrückung eignet sich dann auch als Identifizierungsmerkmal. Allerdings ist dieses Vorgehen aufwändiger und probenbewegungsempfindlicher als die Feststellung von größeren Ortsvariationen. Eine weitere Möglichkeit ist die Nutzung einer unbalancierten chromatischen Dispersion entsprechend US7330270, wobei anschließend aber mehrmals mit unterschiedlichen Dispersionskompensationskoeffizienten numerisch rekonstruiert wird, um unterschiedliche Grade der Verschmierung bei Mess- und Spiegelsignal zu bewirken und festzustellen. Insbesondere bewirkt ein Vorzeichenwechsel bei der Dispersionskorrektur, dass die Verschmierung vom Spiegel- zum Messsignal erfolgt, wodurch ebenfalls eine Identifizierung, beispielsweise durch Bestimmung des zweiten Momentes der Verteilung des zu identifizierenden Signalanteils, erfolgen kann.
Eine weitere vorteilhafte Lösung ist eine grundsätzliche Vermeidung eines unerwünschten Überlappens zwischen Mess- und Spiegelsignal derart, dass die Positionierung der interferometrischen Referenzebene im Bereich zwischen Horn- und Netzhaut ausgeschlossen wird. Eine mögliche Ausführung hierfür ist, dass das Interferometer so gestaltet ist, dass die Referenzebene immer sicher vor der Hornhaut eines in einer Kopfstütze gehaltenen Probanden positioniert wird (Fig. 3b), während der Messbereich ausreichend groß gewählt wird um den Positionierfehler und die Augenlängenbereich abzudecken, insbesondere durch Wahl eines interferometrischen Messbereichs (einseitige A-scan Länge) von mindestens 44mm. Soll die Justage des Patientenauges erleichtert werden, so bietet sich die Wahl eines interferometrischen Messbereichs von mindestens 50mm an.

Günstig ist, dass bei der erfindungsgemäßen Lösung nicht nur ein einzelner Augenbereich, wie Vorderkammer oder Netzhaut, durch einen B-scan erfasst werden kann, sondern dass zusätzliche B-Scan-Informationen aus den jeweils anderen Bereichen gleichzeitig mit gewonnen werden können. Dies ist besonders vorteilhaft, da diese zusätzlichen Informationen nicht nur sehr genaue Messungen des Abstandes der Augenstrukturen innerhalb einzelner A-Scans trotz möglicher Augenbewegungen während der Dauer der B-Scanaufnahme erlauben, sondern auch die Korrektur der B-Scandaten zur Wiedergabe bewegungskorrigierter Bilddaten.
Bevorzugt würde hierzu beispielsweise ein B-Scan einer Netzhautregion aufgenommen, während der B-scan gleichzeitig Bildinformationen der Hornhaut enthält, wie beispielsweise spekulare Reflexe von Hornhautvorder- oder rückfläche. Würden nun axiale Augenbewegungen des Probanden während der B-Scan Aufnahme vorliegen, so würden diese Bewegungen die Verläufe der Hornhaut- und Netzhautstrukturen gemeinsam beeinflussen. Werden nun die Bildinformationen der Hornhaut derart korrigiert, dass sie einer anderweitig durch Messung ermittelten oder angenommenen Form entsprächen, so kann die dazu genutzte Korrektur auch auf die den Netzhautstrukturen entsprechenden Bildinformationen angewendet werden und hierdurch ebenfalls eine bewegungskorrigierte Darstellung erzielt werden. Eine derartige Korrektur kann beispielsweise durch axiale und laterale Verschiebungen der den B-Scan bildenden A-Scans realisiert werden, insbesondere derart, dass die Hornhautstrukturen im Bild beispielsweise minimale Abweichungen von einer zuvor durch Hornhautkeratometrie oder-topographie bestimmten, insbesondere durch stetige Funktionen zu beschreibende Form zeigen.
Eine alternative Korrekturmöglichkeit ist es, den Hornhautverlauf bezüglich seiner scheinbaren lokalen Oberflächenkrümmungen im B-scan zu analysieren und Modulationsfrequenzen abzuleiten, die dann durch Plausibitätsbetrachtungen in Form- und Bewegungsanteile separiert werden können, um anschließend den Bewegungsanteil in den Bilddaten zu reduzieren. Bevorzugt kann dies derart geschehen, dass mindestens eine Grenzfläche der Hornhaut bestimmt wird und deren axiale Positionen relativ zur Lateralablenkung oder auch den korrespondierenden A-Scan-Aufnahmezeitpunkten aufgetragen und durch Fouriertransformation in ein Modulationsfrequenzspektrum überführt wird. Anschließend können Form- und Bewegungsanteil durch geeignet gestaltete Filter gewonnen werden. Die Bewegungsanteile können anschließend durch Fourierrücktransformation und entgegengerichtete Verschiebung der A-Scans in den Bilddaten korrigiert werden, so dass Horn- und Netzhaut, sowie die Vorderkammer bewegungskorrigiert erscheinen. Diese oben genannten Filter für die Modulationsfrequenzen können beispielsweise durch Analyse klinischer Topographiedaten gewonnen werden, wie sie an sich aus der US 7370969 bekannt ist.

## Patentansprüche

1. Vorrichtung zur Swept Source Optical Coherence Domain Reflectometry (SS OCDR) an beweglichen Proben (3), wobei die Proben menschliche Augen sind,
zur Gewinnung von A-Scans mit einem Meßbereich entsprechend der Probengröße,
mit einer durchstimmbaren Laser-Lichtquelle (1) und mindestens einem Empfänger (4) für das aus der Probe zurückgestreute Licht,
wobei die Probe (3) mit einem Messstrahl vom Durchmesser D auf der Probenoberfläche beleuchtet wird, wobei D kleiner 3 mm ist,
wobei die Lichtquelle (1) eine Linienbreite δk<168m⁻¹ aufweist und
wobei die Durchstimmung der Lichtquelle (1) in einer Zeit von τ < 44sec/(D*k₀) um eine Schwerpunktswellenzahl k₀ erfolgt, wobei die Schwerpunktswellenzahl k₀ einer Wellenlänge zwischen 600nm und 1150nm entspricht.

2. Vorrichtung zur SS OCDR nach Anspruch 1, **gekennzeichnet dadurch, dass** die Lichtquelle (1) einen spektralen Durchstimmbereich Δk um die Schwerpunktswellenzahl k₀ von mindestens Δk>18000m⁻¹ aufweist.

3. Vorrichtung zur SS OCDR nach Anspruch 1 oder 2, **gekennzeichnet dadurch, dass** Durchstimmbereich Δk und Linienbreite δk ein Verhältnis Δk / δk größer 360 aufweisen.

4. Vorrichtung zur SS OCDR nach Anspruch 1, 2 oder 3, **gekennzeichnet dadurch, dass** der Quotient aus Durchstimmrate Δk / τ und Laserlinienbreite δk größer als 18 kHz ist.

5. Vorrichtung zur SS OCDR nach Anspruch 1, 2, 3 oder 4, **gekennzeichnet dadurch, dass** eine Digitalisierung des am Empfänger (4) detektierten zurückgestreuten Lichts mit einer Rate von mehr als Δk / (τ*δk) erfolgt.

6. Vorrichtung zur SS OCDR nach Anspruch 1, 2, 3, 4 oder 5, **gekennzeichnet dadurch, dass** die Linienbreite δk der Lichtquelle zwischen 22 und 50 m⁻¹ liegt.

7. Vorrichtung zur SS OCDR nach Anspruch 1, 2, 3, 4, 5 oder 6, **gekennzeichnet dadurch, dass** die Bandbreite des mindestens einem Empfängers (4) größer als 2 * Δk / (τ*δk) und bevorzugt kleiner als 80 Mhz ist.

8. Vorrichtung zur SS OCDR am Auge nach einem der vorigen Ansprüche, **gekennzeichnet dadurch, dass** ein Interferometer (5) mit einem Referenzarm vorgesehen ist, wobei vorzugsweise die Lage von Retina- und Cornea-Signalen im A-Scan und die Laserlinienbreite δk aneinander angepasst sind.

9. Vorrichtung zur SS OCDR am Auge nach einem der vorigen Ansprüche, **gekennzeichnet dadurch, dass** der Messstrahl vor dem Eintritt in das Auge konvergent ist, wobei die Größe der Konvergenz bevorzugt einstellbar oder umschaltbar ist.

10. Vorrichtung zur SS OCDR am Auge nach einem der Ansprüche 1 bis 8, **gekennzeichnet dadurch, dass** der Messstrahl vor dem Eintritt in das Auge kollimiert ist und Mittel (19) zur Umfixierung des Auges vorgesehen sind.

11. Vorrichtung zur SS OCDR am Auge nach einem der vorigen Ansprüche, **gekennzeichnet dadurch, dass** die Vorrichtung dazu eingerichtet ist, dass der Messstrahl außerhalb des Cornea-Apex auf das Auge trifft, wobei vorzugsweise eine Einrichtung zur Positionierung des Messstrahles relativ zum Auge vorgesehen ist, welche insbesondere durch Auswertung des vom Empfänger detektierten Lichts angesteuert werden kann.

12. Vorrichtung zur SS OCDR am Auge nach einem der vorigen Ansprüche, **gekennzeichnet dadurch, dass** in der Durchstimmzeit τ der Lichtquelle ein Photonenfluss von 3 * 10⁸ bis 3 * 10¹³ auf das Auge gerichtet wird.

13. Vorrichtung zur SS OCDR am Auge nach einem der vorigen Ansprüche, **gekennzeichnet dadurch, dass** die Vorrichtung dazu eingerichtet ist, dass die Detektion des von Cornea, Augenlinse und Retina zurückgestreuten Lichtes während einer Durchstimmung der Lichtquelle erfolgt.

14. Verfahren zur SS OCDR am Auge mit einer Vorrichtung nach Anspruch 13, **gekennzeichnet dadurch, dass** der Messstrahl während oder zwischen den einzelnen Durchstimmungen seitlich abgelenkt wird.

15. Verfahren zur SS OCDR am Auge nach Anspruch 14, **gekennzeichnet dadurch, dass** das von der Cornea zurückgestreute und detektierte Licht aus einzelnen Durchstimmungen jeweils hinsichtlich zeitabhängiger Corneapositionen ausgewertet wird, dass diese mit einem erwarteten Verlauf verglichen werden und dass Abweichungen von dem erwarteten Verlauf zur Korrektur von Positionsdaten von Cornea, Augenlinse und/oder Retina genutzt werden.

## Claims

1. Apparatus for Swept Source Optical Coherence Domain Reflectometry (SS OCDR) on movable samples (3), wherein the samples are human eyes,
for obtaining A-scans with a measurement range corresponding to the sample size,
comprising a tunable laser light source (1) and at least one receiver (4) for the light backscattered from the sample,
wherein the sample (3) is illuminated with a measurement beam having the diameter D on the sample surface, wherein D is less than 3 mm,
wherein the light source (1) has a linewidth δk<168m⁻¹, and
wherein the light source (1) is tuned in a time of τ < 44sec/(D*k₀) around a centroid wavenumber k₀, wherein the centroid wavenumber k₀ corresponds to a wavelength of between 600 nm and 1150 nm.

2. Apparatus for SS OCDR according to Claim 1, **characterized in that** the light source (1) has a spectral tuning range Δk around the centroid wavenumber k₀ of at least Δk>18000 m⁻¹.

3. Apparatus for SS OCDR according to Claim 1 or 2, **characterized in that** tuning range Δk and linewidth δk have a ratio Δk / δk of greater than 360.

4. Apparatus for SS OCDR according to Claim 1, 2 or 3, **characterized in that** the quotient of tuning rate Δk / τ and laser linewidth δk is greater than 18 kHz.

5. Apparatus for SS OCDR according to Claim 1, 2, 3 or 4, **characterized in that** the backscattered light detected at the receiver (4) is digitized at a rate of more than Δk / (τ*δk).

6. Apparatus for SS OCDR according to Claim 1, 2, 3, 4 or 5, **characterized in that** the line width δk of the light source is between 22 and 50 m⁻¹.

7. Apparatus for SS OCDR according to Claim 1, 2, 3, 4, 5 or 6, **characterized in that** the bandwidth of the at least one receiver (4) is greater than 2 * Δk / (τ*δk) and preferably less than 80 MHz.

8. Apparatus for SS OCDR on the eye according to any of the preceding claims, **characterized in that** an interferometer (5) having a reference arm is provided, wherein preferably the position of retina and cornea signals in the A-scan and the laser linewidth δk are adapted to one another.

9. Apparatus for SS OCDR on the eye according to any of the preceding claims, **characterized in that** the measurement beam is convergent before entering the eye, wherein the size of the convergence is preferably adjustable or switchable.

10. Apparatus for SS OCDR on the eye according to any of Claims 1 to 8, **characterized in that** the measurement beam is collimated before entering the eye and means (19) for refixation of the eye are provided.

11. Apparatus for SS OCDR on the eye according to any of the preceding claims, **characterized in that** the apparatus is configured to the effect that the measurement beam impinges on the eye outside the cornea apex, wherein a device for positioning the measurement beam relative to the eye is preferably provided, which device can be driven in particular by evaluation of the light detected by the receiver.

12. Apparatus for SS OCDR on the eye according to any of the preceding claims, **characterized in that** in the tuning time τ of the light source a photon flux of 3 * 10⁸ to 3 * 10¹³ is directed onto the eye.

13. Apparatus for SS OCDR on the eye according to any of the preceding claims, **characterized in that** the apparatus is configured to the effect that the light backscattered from cornea, eye lens and retina is detected during a tuning of the light source.

14. Method for SS OCDR on the eye using an apparatus according to Claim 13, **characterized in that** the measurement beam is deflected laterally during or between the individual tunings.

15. Method for SS OCDR on the eye according to Claim 14, **characterized in that** the light backscattered from the cornea and detected from individual tunings is evaluated in each case with regard to time-dependent cornea positions, **in that** the latter are compared with an expected profile, and **in that** deviations from the expected profile are used for correcting position data of cornea, eye lens and/or retina.

## Revendications

1. Dispositif de réflectométrie dans le domaine optique cohérente à source balayée (SS OCDR - Swept Source Optical Coherence Domain Reflectometry) sur des échantillons mobiles (3), les échantillons étant des yeux humains, en vue d'acquérir des écographies A avec une plage de mesure correspondant à la taille de l'échantillon,
comprenant une source de lumière laser (1) accordable et au moins un récepteur (4) pour la lumière rétrodiffusée depuis l'échantillon,
l'échantillon (3) étant éclairé avec un rayon de mesure de diamètre D sur la surface de l'échantillon, D étant inférieur à 3 mm,
la source de lumière (1) possédant une largeur de ligne δk < 168m⁻¹ et
l'accord de la source de lumière (1) étant effectué en un temps τ < 44 s/(D*k₀) autour d'un nombre d'ondes au centre de gravité k₀, le nombre d'ondes au centre de gravité k₀ correspondant à une longueur d'onde comprise entre 600 nm et 1150 nm.

2. Dispositif de SS OCDR selon la revendication 1, **caractérisé en ce que** la source de lumière (1) possède une plage d'accord spectrale Δk d'au moins Δk > 18000 m⁻¹ autour du nombre d'ondes au centre de gravité k₀.

3. Dispositif de SS OCDR selon la revendication 1 ou 2, **caractérisé en ce que** la plage d'accord Δk et la largeur de ligne δk présentent un rapport Δk/δk supérieur à 360.

4. Dispositif de SS OCDR selon la revendication 1, 2 ou 3, **caractérisé en ce que** le quotient du taux d'accord Δk/τ et de la largeur de ligne laser δk est supérieur à 18 kHz.

5. Dispositif de SS OCDR selon la revendication 1, 2, 3 ou 4, **caractérisé en ce qu'**une numérisation de la lumière rétrodiffusée détectée au niveau du récepteur (4) est effectuée avec un taux supérieur à Δk/(τ*8k).

6. Dispositif de SS OCDR selon la revendication 1, 2, 3, 4 ou 5, **caractérisé en ce que** la largeur de ligne δk de la source de lumière est comprise entre 22 et 50 m⁻¹.

7. Dispositif de SS OCDR selon la revendication 1, 2, 3, 4, 5 ou 6, **caractérisé en ce que** la largeur de bande de l'au moins un récepteur (4) est supérieure à 2*Δk/(τ*δk) et de préférence inférieure à 80 MHz.

8. Dispositif de SS OCDR sur l'oeil selon l'une des revendications précédentes, **caractérisé en ce qu'**il existe un interféromètre (5) doté d'un bras de référence, la position des signaux de la rétine et de la cornée dans l'échographie A et la largeur de ligne laser δk étant de préférence adaptées l'une à l'autre.

9. Dispositif de SS OCDR sur l'oeil selon l'une des revendications précédentes, **caractérisé en ce que** le rayon de mesure est convergent avant de pénétrer dans l'oeil, la taille de la convergence étant de préférence réglable ou permutable.

10. Dispositif de SS OCDR sur l'oeil selon l'une des revendications 1 à 8, **caractérisé en ce qu'**avant de pénétrer dans l'oeil, le rayon de mesure est collimaté et il existe des moyens (19) destinés à décaler la fixation de l'oeil.

11. Dispositif de SS OCDR sur l'oeil selon l'une des revendications précédentes, **caractérisé en ce que** le dispositif est conçu pour que le rayon de mesure vienne frapper l'oeil en-dehors du sommet de la cornée, un appareil destiné à positionner le rayon de mesure par rapport à l'oeil étant de préférence présent, lequel peut notamment être commandé en interprétant la lumière détectée par le récepteur.

12. Dispositif de SS OCDR sur l'oeil selon l'une des revendications précédentes, **caractérisé en ce que** pendant le temps d'accord τ de la source de lumière, un flux de photons de 3*10⁸ à 3*10¹³ est dirigé sur l'oeil.

13. Dispositif de SS OCDR sur l'oeil selon l'une des revendications précédentes, **caractérisé en ce que** le dispositif est conçu pour que la détection de la lumière rétrodiffusée par la cornée, le cristallin et la retine s'effectue pendant un accord de la source de lumière.

14. Procédé de SS OCDR sur l'oeil avec un dispositif selon la revendication 13, **caractérisé en ce que** le rayon de mesure est dévié latéralement pendant ou entre les accords individuels.

15. Procédé de SS OCDR sur l'oeil selon la revendication 14, **caractérisé en ce que** la lumière rétrodiffusée par la cornée et détectée issue d'accords individuels est à chaque fois interprétée en ce qui concerne des positions de la cornée en fonction du temps, **en ce que** celles-ci sont comparées à une courbe attendue et **en ce que** les écarts par rapport à la courbe attendue sont utilisés pour la correction de données de position de la cornée, du cristallin et/ou de la rétine.
